# EUROPEAN PATENT SPECIFICATION

(11) **EP 2 251 690 B1**
(45) Date of publication and mention of the grant of the patent: **27.01.2016**
(21) Application number: 09717905.5
(22) Date of filing: 27.02.2009
(51) Int. Cl.: G01N 33/531, G01N 33/543, G01N 33/558

(54) **IMMUNOASSAY METHOD AND KIT**
IMMUNOASSAY-VERFAHREN UND KIT
ESSAI D'IMMUNO-DOSAGE ET KIT

(30) Priority: 06.03.2008 JP 2008056180
(43) Date of publication of application: 17.11.2010
(73) Proprietor: Tanaka Kikinzoku Kogyo K.K., Chiyoda-ku Tokyo 100-6422 (JP)
(72) Inventor: ITO, Daisuke, Hiratsuka-shi Kanagawa 254-0076 (JP); IWAMOTO, Hisahiko, Hiratsuka-shi Kanagawa 254-0076 (JP); MOCHIDUKI, Kazuyoshi, Hiratsuka-shi Kanagawa 254-0076 (JP); TSUGE, Riku, Hiratsuka-shi Kanagawa 254-0076 (JP); KITANI, Yoshiko, Hiratsuka-shi Kanagawa 254-0076 (JP)
(74) Representative: Beckmann, Claus
(86) International application number: PCT/JP2009/054230
(87) International publication number: WO 2009/110577

(56) References cited:
- WO-A2-2005/003732
- WO-A2-2006/065118
- JP-A- 7 325 085
- JP-A- 11 153 600
- JP-A- 2001 289 852
- JP-A- 2002 148 266
- JP-A- 2007 322 310
- JP-T- 2007 524 813

## Description

### TECHNICAL FIELD

The present invention relates to an immunoassay method, and an immunoassay kit comprising a test piece for immunochromatography and a developing solvent for use in the kit.

### BACKGROUND ART

When a target substance, for example, a target substance contained in the blood is to be measured by an immunoassay method, there are the following problems: red corpuscles contained in the blood block the pores of a cell separation matrix, thereby making difficult the flow of an insoluble carrier such as a latex particle or a gold colloidal particle supporting an antibody or the like, the red corpuscles are hemolyzed, thereby interrupting the judgment of a pigment, and hemoglobin readily causes a nonspecific reaction.

To solve these problems, JP-A 2006-177970 teaches that a characteristic property is provided to the cell separation structure of the porous separation matrix of a test piece for immunochromatography or that the porous separation matrix is impregnated with mannitol. However, in this proposal, the complicated structure of the test piece for immunochromatography cannot be avoided, and it is hard to say that the hemolysis problem could be solved completely. It is known from the fact that mannitol impregnated into the porous separation matrix is used as a component of an MPA fluid that mannitol has the function of preventing the hemolysis of red corpuscles. However, when it is used in a concentration sufficiently high to prevent hemolysis completely in the immunoassay method, a new problem may occur that the time for detecting the target substance becomes long or a nonspecific reaction is apt to occur.

WO 2006/065118 A2 discloses an immunochromatographic device and its use. The detection zone may be impregnated with a hydrocarbon that can agglomerate erythrocytes, such as mannitol, see [0038]. The separation zones are provided with a lysis of erythrocytes inhibiting material, such as the detergent Pluronic™ or Tetronic™, see [0040].

### DISCLOSURE OF THE INVENTION

It is therefore an object of the present invention to provide an immunoassay method which has a short detection time and can suppress a nonspecific reaction.

It is another object of the present invention to provide an immunoassay method capable of detecting a target substance contained in the blood in a short detection time by suppressing a nonspecific reaction while hemolysis is prevented by making mannitol and a surfactant coexistent in a measurement system.

It is still another object of the present invention to provide a kit for the above method comprising a test piece immunochromatography and a developing solvent.

Other objects and advantages of the present invention will become apparent from the following description.

According to the present invention, firstly, the above objects and advantages of the present invention are attained by an immunoassay method of immunologically measuring a target substance by using a test piece for immunochromatography which comprises a membrane carrier having a first antibody or a first antigen capable of binding specifically to the target substance immobilized thereon, an insoluble carrier having a second antibody or a second antigen capable of binding specifically to the target substance immobilized thereon, and a porous separation matrix capable of cell separation, wherein the porous separation matrix is impregnated with a surfactant in advance, and the insoluble carrier bound specifically to the target substance through the second antibody or the second antigen is developed with a developing solvent containing mannitol.

According to the present invention, secondly, the above objects and advantages of the present invention are attained by an immunoassay method of immunologically measuring a target substance by using a test piece for immunochromatography which comprises a membrane carrier having a first antibody or a first antigen capable of binding specifically to the target substance immobilized thereon, an insoluble carrier having a second antibody or a second antigen capable of binding specifically to the target substance immobilized thereon, and a porous separation matrix capable of cell separation, wherein the porous separation matrix is impregnated with a surfactant in advance, and the insoluble carrier bound specifically to the target substance through the second antibody or the second antigen is developed with a developing solvent containing both mannitol and a surfactant.

According to the present invention, in the third place, the above objects and advantages of the present invention are attained by an immunoassay kit comprising a combination of (1) a test piece for immunochromatography, which comprises a membrane carrier having a first antibody or a first antigen capable of binding specifically to the target substance immobilized thereon, an insoluble carrier having a second antibody or a second antigen capable of binding specifically to the target substance immobilized thereon, and a porous separation matrix capable of cell separation and impregnated with a surfactant and (2) a developing solvent containing mannitol, which is used to develop the insoluble carrier bound specifically to the target substance through the second antibody or the second antigen.

According to the present invention, in the fourth place, the above objects and advantages of the present invention are attained by an immunoassay kit comprising a combination of (1) a test piece for immunochromatography, which comprises a membrane carrier having a first antibody or a first antigen capable of binding specifically to the target substance immobilized thereon, an insoluble carrier having a second antibody or a second antigen capable of binding specifically to the target substance immobilized thereon and a porous separation matrix capable of cell separation and impregnated with a surfactant and (2) a developing solvent containing both mannitol and a surfactant, which is used to develop the insoluble carrier bound specifically to the target substance through the second antibody or the second antigen.

It is considered that the technical feature of the present invention is obtained through the coexistence of mannitol and the surfactant in the measurement system.

Other objects and advantages of the present invention will become apparent from the following description.

### BRIEF DESCRIPTION OF THE DRAWINGS

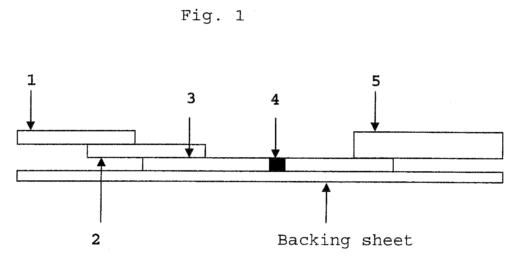

Fig. 1 is a schematic diagram of a test piece for immunochromatography.

### BEST MODE FOR CARRYING OUT THE INVENTION

The surfactant used in the immunoassay method of the present invention is not particularly limited if it is a surfactant used for biochemistry. The surfactant is preferably a surfactant having an HLB of 8 to 20, more preferably a nonionic surfactant having an HLB of 8 to 20. Examples of the surfactant include TritonX-100 (trade name) : (polyethylene glycol mono-p-isooctylphenyl ether), Tween 20: (polyoxyethylene sorbitan monolaurate), Tween 80: (polyoxyethylene sorbitan monooleate), Nonidet P-40: Nonidet P-40 and ZWITTERGENT 3-14: (n-tetradecyl-N,N-dimethyl-3-ammonio-1-propanesulfonate), CHAPS: (3-[(3-colamidepropyl)dimethylammonio]-1-propanesulfonate, and sodium dodecylsulfate (SDS). These surfactants may be used alone or in combination of two or more.

The technique of immunochromatography is known and its principle is typically shown in Fig. 1.

A sample of a target substance to be inspected is dropped on a material addition site 1 in Fig. 1.

The sample addition site 1 is composed of a test piece for immunochromatography which comprises a porous separation matrix capable of cell separation. The porous separation matrix is preferably impregnated with a surfactant before the sample is dropped. The method of impregnating the porous separation matrix with the surfactant is not particularly limited. For example, after the matrix is immersed in the surfactant solution, it is preferably dried by heating.

The specimen to be inspected in the present invention is not particularly limited if it contains a cell or a blood corpuscle and is, for example, blood, urine or a spinal fluid.

Examples of the target substance to be detected from the specimen include virus surface antigens such as HBs antigen, tumor markers such as PSA, CEA and AFP, and immunoglobulins such as anti-HIV antibody, anti-HBV antibody, anti-HCV antibody, anti-mite allergen antibody and anti-cedar pollen allergen antibody. The present invention is not limited to these. These specimens are preferably sampled by a method in which cells and blood corpuscles are not destroyed.

The sample dropped on the sample addition site 1 is developed in the direction of an absorption site 5 by a chromatography medium 3 through a labeled substance holding site 2 supporting an insoluble carrier having an antibody or an antigen capable of binding specifically to the target substance (to be referred to as "second antibody" or "second antigen", respectively) immobilized thereon. An antibody or an antigen capable of binding specifically to the target substance (to be referred to as "first antibody" or "first antigen", respectively) is immobilized on a detection site 4. A carrier having the detection site 4, for developing the target substance by the chromatography medium 3, is a membrane carrier.

In the present invention, a developing solvent containing mannitol is used as the above chromatography medium. Mannitol is preferably contained in the developing solvent in an amount of 1 to 15 wt%. By using the developing solvent having the above mannitol concentration, the target substance can be detected in a short period of time while hemolysis is prevented and a nonspecific reaction is suppressed. The solvent having the above mannitol concentration is not known as a developing solvent for immunochromatography.

When the target substance is contained in the specimen, the target substance reacts with the second antibody or the second antigen, a composite of these is captured by the first antigen or the first antibody at the detection site 4 and a colored band appears. Based on the color of the band which appears at 4, the amount of the target substance contained in the specimen can be roughly known.

The second antigen or the second antibody used at the labeled substance holding site 2 and the first antigen or the first antibody used at the detection site 4 should bind to different sites of the target substance.

As the antibody may be used an antibody which binds specifically to the target substance. Examples of the animal species producing the antibody include humans, mice, rats, rabbits, goats and horses each of which has a predetermined range of immunoglobulins. The antibody may be any one of IgG, IgM, IgA, IgE and IgD, or monoclonal antibody, polyclonal antibody or a fragment thereof (having binding ability to the antigen; such as H chain, L chain, Fab or F(ab')₂).

Specific examples of the antibody include anti-PSA antibody, anti-AFP antibody, anti-CEA antibody, anti-HBs antibody, anti-IgG antibody, anti-human IgE antibody and fractions thereof (having binding ability to the antigen; such as F(ab)'₂ and Fab').

As the antigen may be used an antigen which binds specifically to the target substance. Examples of the antigen include Hbs antigen, HCV antigen, mite allergen and cedar pollen allergen.

Further, examples of the insoluble carrier for labeling the antigen and the antibody include metal colloidal particles such as gold colloidal particles, non-metal colloidal particles such as selenium colloidal particles, colored resin particles such as latex particles, and insoluble particulate substances such as dye colloidal particles and colored liposome. Examples of the enzyme for labeling the antigen and the antibody include peroxidase, alkali phosphatase and glucose oxidase.

The membrane carrier for chromatography media is not particularly limited if it can absorb and fluidize the sample specimen by a capillary phenomenon. The membrane carrier is selected from the group consisting of nitrocellulose, cellulose acetate, nylon, polyether sulfone, polyvinyl alcohol, polyester, glass fiber, polyolefin, cellulose and artificial polymer comprising mixture fibers thereof.

In the test piece for immunochromatography used in the method of the present invention, the porous separation matrix is impregnated with preferably a surfactant, more preferably a surfactant having an HLB of 8 to 20 in advance. Based on this characteristic feature, there is provided a test piece for immunochromatography which comprises a membrane carrier having a first antibody or a first antigen capable of binding specifically to a target substance immobilized thereon, an insoluble carrier having a second antibody or a second antigen capable of binding specifically to the target substance immobilized thereon, and a porous separation matrix capable of cell separation and impregnated with a surfactant.

According to the present invention, there is also provided an immunoassay kit comprising a combination of the above test piece for immunochromatography of the present invention and a developing solvent containing preferably 1 to 15 wt% of mannitol as a kit for carrying out the above method of the present invention. There is further provided a kit for carrying out the above method of the present invention, wherein a preferred developing solvent containing a surfactant and mannitol used in the above immunoassay method is contained.

### Examples

The following examples are provided to further illustrate the present invention. The present invention is not limited by the examples.

### Example 1

### (1) formation of judging part on chromatography medium

A nitrocellulose sheet (HF120 (trade name) of Millipore Co., Ltd. , 300 mm x 25 mm) was used as the membrane carrier (membrane). A mouse-derived anti-carcinoma prostate specific antigen (PSA) monoclonal antibody (first antibody) was diluted with a phosphoric acid buffer solution (pH of 7.4) containing 5 wt% of isopropyl alcohol to a concentration of 1.0 mg/ml. 150 µl of this solution was applied to the membrane to a width of 1 mm and dried at 50°C for 30 minutes. After drying, the membrane was immersed in 100 ml of a phosphoric acid buffer solution (pH of 7.4) containing 0.5 wt% of casein (manufactured by Wako Pure Chemical Industries, Ltd.) at room temperature for 30 minutes to carry out blocking. After blocking, the membrane was rinsed with a phosphoric acid buffer solution (pH of 7.4) containing 0.05 wt% of Tween 20 and dried at room temperature for one night to prepare a chromatography medium (membrane carrier having the first antigen immobilized thereon).

### (2) preparation of labeled substance solution

0.1 ml of a mouse-derived anti-PSA monoclonal antibody (second antibody) diluted with a phosphoric acid buffer solution (pH of 7.4) to a concentration of 0.1 mg/ml was added to 0.5 ml of a gold colloidal dispersion (LC40nm of Tanaka Kikinzoku Kogyo Co., Ltd.) and left to stand at room temperature for 10 minutes. Then, 0.1 ml of a phosphoric acid buffer solution (pH of 7.4) containing 10 wt% of bovine serum albumin (to be abbreviated as BSA) was added to the resulting mixture and left to stand at room temperature for 10 minutes. After it was fully stirred, it was centrifuged at 8,000 x g for 15 minutes to remove the supernatant, and 0.1 ml of a phosphoric acid buffer solution (pH of 7.4) containing 1 wt% of BSA was added. The labeled substance solution was prepared by the above procedure.

### (3) preparation of test piece for immunochromatography

A solution prepared by adding 300 µl of a 10 wt% trehalose aqueous solution and 1.8 ml of distilled water to 300 µl of the labeled substance solution prepared above was added to a glass fiber pad measuring 15 mm x 300 mm (manufactured by Millipore Co., Ltd.) uniformly, and the pad was dried with a vacuum drier to prepare a label holding member (insoluble carrier having the second antibody immobilized thereon).

A blood corpuscle separation membrane (Pole Co. , Ltd.: 300 mm x 30 mm) was used as a sample pad (porous separation matrix). 3 ml of a 20 mM Tris-HCl buffer solution (pH of 8.0) containing 0.1 wt% of Triton (registered trademark) X-100 (HLB = 13) was absorbed into this sample pad uniformly which was then dried at 40°C for 1 hour.

The chromatography medium prepared above, the labeled substance holding member, the above sample pad for use in a portion to which the sample was to be added and an absorption pad for absorbing the developed sample and the labeled substance were put on a substrate composed of a backing sheet. The resulting laminate was cut to a width of 5 mm by a cutting machine to prepare a test piece for immunochromatography.

### (4) preparation of developing solvent

D-mannitol (manufactured by Wako Pure Chemical Industries, Ltd.) was added to 10 ml of a HEPES buffer solution (pH of 8.0) containing 1 wt% of BSA and 150 mM NaCl to a concentration of 10 wt% so as to prepare a developing solvent.

### (5) measurement

The existence of PSA in the blood was checked by the following method using the test piece for immunochromatography prepared above.

That is, a negative specimen having a PSA concentration in the blood of less than 0.1 ng/ml and a positive specimen having a PSA concentration of 4 ng/ml were used as specimens to be inspected, 25 µl of each specimen to be inspected was placed on the sample pad of the test piece for immunochromatography, 100 µl of the developing solvent was placed on the sample pad to develop it, and the specimen was checked with the eye after 15 minutes. The specimen was evaluated as "+" when a red test line could be seen, as "±" when a red line could be seen but its color was very pale, as "-" when the red line could not be seen and as "impossible to judge" when the specimen could not be measured based on hemolysis. The results are shown in Table 1. The existence of hemolysis is shown in Table 2.

### Example 2

The measurement was made in the same manner as in Example 1 except that the surfactant to be absorbed into the sample pad was changed to Tween20 (HLB = 17) from Triton (registered trademark) X-100. The results are shown in Table 1. The existence of hemolysis is shown in Table 2.

### Example 3

The measurement was made in the same manner as in Example 1 except that the surfactant to be absorbed into the sample pad was changed to Adecanol NK-4 (HLB = 9) from Triton (registered trademark) X-100. The results are shown in Table 1. The existence of hemolysis is shown in Table 2.

### Example 4

The measurement was made in the same manner as in Example 1 except that the concentration of D-mannitol in the developing solvent was changed to 3 wt%. The results are shown in Table 1. The existence of hemolysis is shown in Table 2.

### Example 5

The measurement was made in the same manner as in Example 1 except that the concentration of D-mannitol in the developing solvent was changed to 12 wt%. The results are shown in Table 1. The existence of hemolysis is shown in Table 2.

### Example 6

The measurement was made in the same manner as in Example 1 except that Triton X-100 was added to the developing solvent to a concentration of 0.05 wt%. The results are shown in Table 1. The existence of hemolysis is shown in Table 2.

Even when the measurement was made in the same manner as in Example 6 except that the surfactant was not absorbed into the sample pad, the same results were obtained.

### Comparative Example 1

The measurement was made in the same manner as in Example 1 except that D-mannitol contained in the developing solvent was changed to glycerol. The results are shown in Table 1. The existence of hemolysis is shown in Table 2.

**Table 1**

| Specimen to be inspected | Ex.1 | Ex.2 | Ex.3 | Ex.4 | Ex.5 | Ex.6 | C.Ex.1 |
|---|---|---|---|---|---|---|---|
| Negative specimen | - | - | - | - | - | - | Impossible to judge |
| Positive specimen | + | + | + | + | + | + | Impossible to judge |

| | | | | | | | |
|---|---|---|---|---|---|---|---|
| Ex.: Example C.Ex.: Comparative Example | | | | | | | |

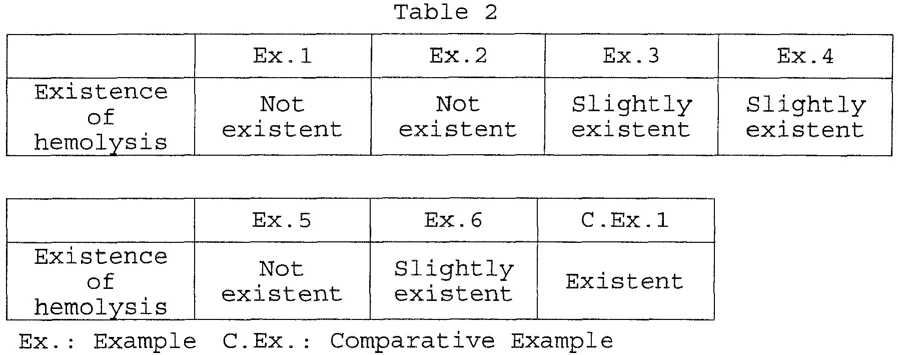

### Example 7

The measurement was made in the same manner as in Example 1 except that the antibody to be applied to the membrane was changed to a mouse derived anti-cancer embryonal antigen (CEA) monoclonal antibody (first antibody), the antibody to be immobilized on the gold colloid was changed to a mouse derived anti-CEA monoclonal antibody (second antibody), the negative specimen to be measured was blood having a CEA concentration of less than 1 ng/ml, and the positive specimen was blood having a CEA concentration of 10 ng/ml as the specimens used in the measurement. The results including the existence of hemolysis are shown in Table 3.

**Table 3**

| Specimens to be inspected | Example 7 |
|---|---|
| Negative specimen | - |
| Positive specimen | + |
| Existence of hemolysis | Not existent |

According to the present invention, there can be provided an immunoassay method which is capable of detecting a target substance in a short period of time and enables accurate detection by suppressing a nonspecific reaction, and a kit and a developing solvent for use in the method.

## Claims

1. An immunoassay method of immunologically measuring a target substance by using a test piece for immunochromatography which comprises a membrane carrier having a first antibody or a first antigen capable of binding specifically to the target substance immobilized thereon, an insoluble carrier having a second antibody or a second antigen capable of binding specifically to the target substance immobilized thereon, and a porous separation matrix capable of cell separation, wherein
the porous separation matrix is impregnated with a surfactant in advance, and the insoluble carrier bound specifically to the target substance through the second antibody or the second antigen is developed with a developing solvent containing mannitol.

2. An immunoassay method of immunologically measuring a target substance by using a test piece for immunochromatography which comprises a membrane carrier having a first antibody or a first antigen capable of binding specifically to the target substance immobilized thereon, an insoluble carrier having a second antibody or a second antigen capable of binding specifically to the target substance immobilized thereon, and a porous separation matrix capable of cell separation, wherein the porous separation matrix is impregnated with a surfactant in advance, and the insoluble carrier bound specifically to the target substance through the second antibody or the second antigen is developed with a developing solvent containing both mannitol and a surfactant.

3. The immunoassay method according to any one of claims 1 to 2, wherein the HLB of the surfactant is in the range of 8 to 20.

4. The immunoassay method according to any one of claims 1 to 2, wherein the developing solvent contains 1 to 15 wt% of mannitol.

5. An immunoassay kit comprising a combination of (1) a test piece for immunochromatography, which comprises a membrane carrier having a first antibody or a first antigen capable of binding specifically to the target substance immobilized thereon, an insoluble carrier having a second antibody or a second antigen capable of binding specifically to the target substance immobilized thereon, and a porous separation matrix capable of cell separation and impregnated with a surfactant and (2) a developing solvent containing mannitol, which is used to develop the insoluble carrier bound specifically to the target substance through the second antibody or the second antigen.

6. An immunoassay kit comprising a combination of (1) a test piece for immunochromatography, which comprises a membrane carrier having a first antibody or a first antigen capable of binding specifically to the target substance immobilized thereon, an insoluble carrier having a second antibody or a second antigen capable of binding specifically to the target substance immobilized thereon and a porous separation matrix capable of cell separation and impregnated with a surfactant and (2) a developing solvent containing both mannitol and a surfactant, which is used to develop the insoluble carrier bound specifically to the target substance through the second antibody or the second antigen.

7. The immunoassay kit according to any one of claims 5 to 6, wherein the HLB of the surfactant is in the range of 8 to 20.

8. The immunoassay kit according to any one of claims 5 to 6, wherein the developing solvent contains 1 to 15 wt% of mannitol.

## Patentansprüche

1. Immunassay-Verfahren zur immunologischen Messung einer Zielsubstanz durch Verwendung eines Teststücks für Immunchromatographie, das einen Membranträger, der einen ersten Antikörper oder ein erstes Antigen, der/das fähig ist, spezifisch an die Zielsubstanz zu binden, daran immobilisiert hat, einen unlöslichen Träger, der einen zweiten Antikörper oder ein zweites Antigen, der/das fähig ist, spezifisch an die Zielsubstanz zu binden, daran immobilisiert hat, und eine poröse Trennungsmatrix, die zur Zelltrennung geeignet ist, umfasst, wobei
die poröse Trennungsmatrix vorab mit einem Surfactant imprägniert wird und der unlösliche Träger, der spezifisch an die Zielsubstanz durch den zweiten Antikörper oder das zweite Antigen gebunden ist, mit einem Entwicklungslösungsmittel, das Mannit enthält, entwickelt wird.

2. Immunassay-Verfahren zur immunologischen Messung einer Zielsubstanz durch Verwendung eines Teststücks für Immunchromatographie, das einen Membranträger, der einen ersten Antikörper oder ein erstes Antigen, der/das fähig ist, spezifisch an die Zielsubstanz zu binden, daran immobilisiert hat, einen unlöslichen Träger, der einen zweiten Antikörper oder ein zweites Antigen, der/das fähig ist, spezifisch an die Zielsubstanz zu binden, daran immobilisiert hat, und eine poröse Trennungsmatrix, die zur Zelltrennung fähig ist, umfasst, wobei die poröse Trennungsmatrix vorab mit einem Surfactant imprägniert wird, und der unlösliche Träger, der spezifisch durch den zweiten Antikörper oder das zweite Antigen an die Zielsubstanz gebunden ist, mit einem Entwicklungslösungsmittel, das sowohl Mannit als auch ein Surfactant enthält, entwickelt wird.

3. Immunassay-Verfahren gemäß einem der Ansprüche 1 bis 2, wobei der HLB-Wert des Surfactants im Bereich von 8 bis 20 ist.

4. Immunassay-Verfahren gemäß einem der Ansprüche 1 bis 2, wobei das Entwicklungslösungsmittel 1 bis 15 Gew.-% Mannit enthält.

5. Immunassay-Kit, umfassend eine Kombination aus (1) einem Teststück für Immunchromatographie, das einen Membranträger, der einen ersten Antikörper oder ein erstes Antigen, der/das fähig ist, spezifisch an die Zielsubstanz zu binden, daran immobilisiert hat, einen unlöslichen Träger, der einen zweiten Antikörper oder ein zweites Antigen, der/das fähig ist, spezifisch an die Zielsubstanz zu binden, daran immobilisiert hat, und eine poröse Trennungsmatrix, die zur Zelltrennung fähig ist und mit einem Surfactant imprägniert ist, umfasst, und (2) einem Entwicklungslösungsmittel, das Mannit enthält, das verwendet wird, um den unlöslichen Träger, der spezifisch an die Zielsubstanz durch den zweiten Antikörper oder das zweite Antigen gebunden ist, zu entwickeln.

6. Immunassay-Kit, umfassend eine Kombination aus (1) einem Teststück für Immunchromatographie, das einen Membranträger, der einen ersten Antikörper oder ein erstes Antigen, der/das fähig ist, spezifisch an die Zielsubstanz zu binden, daran immobilisiert hat, einen unlöslichen Träger, der einen zweiten Antikörper oder ein zweites Antigen, der/das fähig ist, spezifisch an die Zielsubstanz zu binden, daran immobilisiert hat, und eine poröse Trennungsmatrix, die zur Zelltrennung fähig ist und mit einem Surfactant imprägniert ist, umfasst, und (2) einem Entwicklungslösungsmittel, das sowohl Mannit als auch ein Surfactant enthält, das verwendet wird, um den unlöslichen Träger, der spezifisch an die Zielsubstanz durch den zweiten Antikörper oder das zweite Antigen gebunden ist, zu entwickeln.

7. Immunassay-Kit gemäß einem der Ansprüche 5 bis 6, wobei der HLB-Wert des Surfactants im Bereich von 8 bis 20 liegt.

8. Immunassay-Kit gemäß einem der Ansprüche 5 bis 6, wobei das Entwicklungslösungsmittel 1 bis 15 Gew.-% Mannit enthält.

## Revendications

1. Procédé d'immunodosage pour mesurer immunologiquement une substance cible à l'aide d'une pièce d'essai pour immunochromatographie qui comprend un support de type membrane portant un premier anticorps ou un premier antigène capable de se lier spécifiquement à la substance cible immobilisé sur sa surface, un support insoluble portant un second anticorps ou un second antigène capable de se lier spécifiquement à la substance cible immobilisé sur sa surface, et une matrice de séparation poreuse capable de séparer des cellules, dans lequel la matrice de séparation poreuse est imprégnée de tensioactif au préalable, et le support insoluble lié spécifiquement à la substance cible par l'intermédiaire du second anticorps ou du second antigène est révélé à l'aide d'un agent de révélation contenant du mannitol.

2. Procédé d'immunodosage pour mesurer immunologiquement une substance cible à l'aide d'une pièce d'essai pour immunochromatographie qui comprend un support de type membrane portant un premier anticorps ou un premier antigène capable de se lier spécifiquement à la substance cible immobilisé sur sa surface, un support insoluble portant un second anticorps ou un second antigène capable de se lier spécifiquement à la substance cible immobilisé sur sa surface, et une matrice de séparation poreuse capable de séparer des cellules, dans lequel la matrice de séparation poreuse est imprégnée de tensioactif au préalable, et le support insoluble lié spécifiquement à la substance cible par l'intermédiaire du second anticorps ou du second antigène est révélé à l'aide d'un agent de révélation contenant à la fois du mannitol et un tensioactif.

3. Procédé d'immunodosage selon l'une quelconque des revendications 1 à 2, dans lequel la valeur BHL du tensioactif est dans la plage de 8 à 20.

4. Procédé d'immunodosage selon l'une quelconque des revendications 1 à 2, dans lequel le solvant de révélation contient de 1 à 15 % en poids de mannitol.

5. Kit d'immunodosage comprenant une combinaison de (1) une pièce d'essai pour immunochromatographie, qui comprend un support de type membrane portant un premier anticorps ou un premier antigène capable de se lier spécifiquement à la substance cible immobilisé sur sa surface, un support insoluble portant un second anticorps ou un second antigène capable de se lier spécifiquement à la substance cible immobilisé sur sa surface, et une matrice de séparation poreuse capable de séparer des cellules et imprégnée de tensioactif et (2) un agent de révélation contenant du mannitol, qui est utilisé pour révéler le support insoluble lié spécifiquement à la substance cible par l'intermédiaire du second anticorps ou du second antigène.

6. Kit d'immunodosage comprenant une combinaison de (1) une pièce d'essai pour immunochromatographie, qui comprend un support de type membrane portant un premier anticorps ou un premier antigène capable de se lier spécifiquement à la substance cible immobilisé sur sa surface, un support insoluble portant un second anticorps ou un second antigène capable de se lier spécifiquement à la substance cible immobilisé sur sa surface, et une matrice de séparation poreuse capable de séparer des cellules et imprégnée de tensioactif et (2) un agent de révélation contenant à la fois du mannitol et un tensioactif, qui est utilisé pour révéler le support insoluble lié spécifiquement à la substance cible par l'intermédiaire du second anticorps ou du second antigène.

7. Kit d'immunodosage selon l'une quelconque des revendications 5 à 6, dans lequel la valeur BHL du tensioactif est dans la plage de 8 à 20.

8. Kit d'immunodosage selon l'une quelconque des revendications 5 à 6, dans lequel le solvant de révélation contient de 1 à 15 % en poids de mannitol.
